# EUROPÄISCHE PATENTSCHRIFT

(11) **EP 1 512 011 B1**
(45) Veröffentlichungstag und Bekanntmachung des Hinweises auf die Patenterteilung: **27.04.2011**
(21) Anmeldenummer: 03757028.0
(22) Anmeldetag: 05.06.2003
(51) Int. Cl.: G01N 33/543, G01N 33/548

(54) **FESTPHASENSUBSTRAT ZUR IMMOBILISIERUMG VON BIOMOLEK LEN**
SOLID PHASE SUBSTRATE FOR IMMOBILIZING BIOMOLECULES
SUBSTRAT EN PHASE SOLIDE DESTINE A L'IMMOBILISATION DE BIOMOLECULES

(30) Priorität: 10.06.2002 DE 10225501
(43) Veröffentlichungstag der Anmeldung: 09.03.2005
(73) Patentinhaber: QIAGEN North American Holdings, Inc., Germantown, MD 20874 (US)
(72) Erfinder: PAPRA, Alexander, 22159 Hamburg (DE); KOLZAU, Thomas, 22399 Hamburg (DE); KÖPPEN, Barbara, 22523 Hamburg (DE)
(74) Vertreter: Schneider, Jürgen M.
(86) Internationale Anmeldenummer: PCT/EP2003/005909
(87) Internationale Veröffentlichungsnummer: WO 2003/104809

(56) Entgegenhaltungen:
- WO-A-03/050276
- US-A- 5 196 536
- US-A- 5 403 750
- US-A- 5 624 831
- CORDONE LORENZO ET AL: "A reduction of protein specific motion in co-ligated myoglobin embedded in a trehalose glass" EUROPEAN BIOPHYSICS JOURNAL, Bd. 27, Nr. 2, 1998, Seiten 173-176, XP002258298 ISSN: 0175-7571
- NGUYEN VU KHUE ET AL: "Stabilization of dry immobilized acetylcholinesterase on nitrocellulose membrane for rapid colorimetric screening of its inhibitors in water biological fluids" ANALYTICAL LETTERS, Bd. 31, Nr. 14, November 1998 (1998-11), Seiten 2457-2473, XP009019271 ISSN: 0003-2719 in der Anmeldung erwähnt

## Beschreibung

Die Erfindung bezieht sich auf ein Festphasensubstrat zur Immobilisierung von Biomolekülen nach dem Oberbegriff des Anspruchs 1 sowie auf ein Verfahren zur Immobilisierung von Biomolekülen in einer Probe nach dem Oberbegriff des Anspruchs 8.

Die Immobilisierung von Biomolekülen an Festphasensubstraten ist z.B. erforderlich bei der Herstellung von Arrays, z.B. Protein-Arrays, Enzymmembranen, mit Proteinen beschichteten Reaktionsgefäßen etc.

Gattungsgemäße insbesondere für Enzymmembranen geeignete Festphasensubstrate bestehen z.B. aus Nitrozellulose, die aufgrund ihres geringen Preises, ihrer Flexibilität und ihrer guten Bindungseigenschaften insbesondere für Proteine verwendet wird. Die für die Bindungseigenschaften von Nitrozellulose gegenüber Biomolekülen verantwortlichen Mechanismen sind unbekannt. Sie beruhen jedoch vermutlich auf der Bildung von Wasserstoffbrücken-Bindungen sowie auf hydrophoben und elektrostatischen Wechselwirkungen. Die Adsorption erfolgt in der Praxis z.B. durch einfache Inkubation des Substrats in einer Biomoleküle enthaltende Lösung und anschließende Trocknung.

Dabei ist es bekannt (Nguyen VK et al. 1998, Stabilization of dry immobilized acetylcholinesterase on nitrocellulose membrane for rapid colorimetric screening of its inhibitors in water and biological fluids, Analytical letters 31(14), 2457), die Biomoleküle in Anwesenheit von Polyolen wie z.B. Trehalose zu immobilisieren, um eine Denaturierung der Biomoleküle während der Trocknung zu verhindern. Ziel ist, die an dem Substrat immobilisierten Biomoleküle weitestgehend in ihrer natürlichen Konformation zu erhalten, dergestalt, daß z.B. immobilisierte Enzyme ihre katalytische Aktivität beibehalten oder Antikörper ihre spezifischen immunologischen Eigenschaften bewahren. Die im jeweiligen Falle verwendeten Polyole werden auch als Schutzsubstanzen bezeichnet.

Dabei können die Polyole sowohl gleichzeitig mit als auch nach den zu immobilisierenden Biomolekülen auf die Nitrozelluosemembran adsorbiert werden. Da Polyole mit Ihren OH-Gruppen ähnliche funktionelle Gruppen aufweisen wie viele Biomoleküle (z.B die alkoholischen Aminosäuren Tyrosin und Serin), kann angenommen werden, daß sie ähnlich wie die Biomoleküle auf der Oberfläche der Nitrozellulosemembran unspezifisch adsorbieren.

Ein Nachteil bei diesen gattungsgemäßen Substraten ist, daß die auf dem Substrat adsorbierten, nicht kovalent gebundenen Polyole bei der späteren Verwendung des Substrats in Lösung gehen und den anschließenden Versuch beeinträchtigen können. Es ist daher in der Regel erforderlich, das getrocknete Substrat vor einer weiteren Anwendung bzw. Aufarbeitung sorgfältig zu waschen.

Aufgabe der Erfindung ist es, ein Substrat und ein Verfahren zu schaffen, mit denen sich dem oben beschriebenen Nachteil begegnen läßt.

Gelöst wird die Aufgabe mit einem Festphasensubstrat mit einem Bindungsbereich, der zur Immobilisierung von Biomolekülen geeignet ist, wobei des Substrat die kennzeichnenden Merkmale des Anspruchs 1 aufweist, sowie mit einem Verfahren, das die kennzeichnenden Merkmale des Anspruches 8 aufweist.

Das erfindungsgemäße Festphasensubstrat weist gemäß Anspruch 1 im Bindungsbereich reaktive Bindungsstellen auf, wobei an einem Teil davon Polyole vorkonfektioniert mittels kovalenter Bindungen immobilisiert sind. Geeignete Substrate können z.B. aus Kunststoff aber natürlich auch aus Glas bestehen. In der Regel handelt es sich um flächige Substrate, z.B. Slides oder Objekträger. Denkbar sind selbstverstänlich auch andere Substrate z.b. die Wände von Reaktionsgefäßen oder dergleichen. Grundsätzlich sollen alle im Rahmen der hier angesprochenen Immobilisierung von Biomolekülen sinnvolle Substrate durch die Erfindung abgedeckt werden.

Bei Verwendung des erfindungsgemäßen Substrates erübrigt sich die bei gattungsgemäßen Substraten erforderliche separate Adsorption des Polyols. Es müssen also während des gesamten weiteren Verarbeitungsprozesses keine Polyole mehr zugegeben werden. Überdies muß das Substrat vor einer weiteren Verwendung nicht gewaschen werden, da das vorliegende Polyol kovalent gebunden und nicht nur adsorbiert ist, und daher eine Beeinträchtigung eines anschließenden Versuchs durch in Lösung gehende Polyole nicht zu befürchten ist. Ein solches, vorkonfektioniert mit kovalent gebundenen Polyolen beschichtetes Festphasensubstrat eignet sich insbesondere für das Bespotten mit Protein- oder Nukleinsäuresonden bzw. -proben, und ist daher besonders gut für die Herstellung von Bioarrays geeignet.

In einer bevorzugten Ausgestaltung ist vorgesehen, daß der Bindungsbereich zur Immobilisierung von Proteinen ausgelegt ist. Die reaktiven Bindungsstellen im Bindungsbereich müssen daher für die kovalente Bindung von Proteinen geeignete funktionelle Gruppen aufweisen. Diese können z.B. esteraktiv sein, so daß sie mit Aminogruppen kovalente Bindungen eingehen können. Denkbar sind selbstverständlich auch andere Bindungsstellen. Unter Bindungsstellen sollen im Rahmen der Erfindung im wesentlichen im Bindungsbereich an das Substrat gekoppelte funktionelle Gruppen verstanden werden, die in der Lage sind mit den jeweiligen Biomolekülen und den Polyolen eine Bindung einzugehen.

In weiteren erfindungsgemäßen Ausführungsformen kann der Bindungsbereich so ausgelegt sein, daß er sich zur Bindung anderer Biomoleküle, wie z.B. Zucker, Lipide oder Nukleinsäuren eignet. Hierfür eignen sich die üblichen dem Fachmann bekannten Bindungsstellen, auf die hier nicht näher eingegangen werden soll.

In einer besonders bevorzugten Ausgestaltung weist das Substrat an seine Oberfläche gekoppelte Polymerketten auf. Dabei ist vorgesehen, daß die an dem Substrat angeordneten Polymerketten die erforderlichen reaktiven Bindungsstellen zur Kopplung der Biomoleküle tragen. In diesem Fall sind an den Polymerketten ebenfalls die zur Stabilisierung der immobilisierten Biomoleküle erforderlichen Polyole gebunden.

Die Polymerketten können unvernetzt mit jeweils ihrem einen Ende an die Oberfläche des Substrats gekoppelt sein und mit dem anderen Ende davon abstehen. Denkbar sind aber auch Vernetzungen der Polymere untereinander in unterschiedlichen Graden, bis hin zu einem Hydrogel. Die Verwendung solcher Polymerketten erhöht die aktive Oberfläche des Substrats und gestattet so die Bindung einer größeren Menge von Polyolen und/oder Biomolekülen.

In einer bevorzugten Ausgestaltung ist außerdem vorgesehen, daß in den Polymerketten Polyethylengykol gebunden ist. Solche PEG-enthaltenden Polymere schirmen die Substratoberfläche z.B. gegen unspezifische, nicht kovalente Absorption anderer Proteine ab.

Bei der Herstellung der vorkonfektionierten Substrate können im Rahmen der Erfindung Polyole, z.B. Mono-, Di- oder Trisaccharide als Schutzsubstanzen eingesetzt werden. Geeignet sind z.B. Maltose, Saccharose, Raffinose oder Glukose. Grundsätzlich sind alle Substanzen geeignet, die über OH-Gruppen an eine aktivierte Oberfläche eines Substrats gekoppelt werden können und die in der Lage sind, die dreidimensionale Konformation von Biomolekülen, z.B. Proteinen zu stabilisieren. Ein besonders bevorzugt eingesetztes Polyol ist Trehalose.

In einer bevorzugten Ausführungsform ist vorgesehen, daß das Festphasensubstrat ein Biochip, ein Enzymchip, ein Proteinarray, eine Filtermembran, ein Microbead, ein Reaktionsgefäß, ein Mikrokanalsystem, ein Durchflußschlauchsystem, eine Pipettenspitze oder eine Durchflußkanüle ist.

Die Erfindung soll jedoch auch Verfahren zur Immobilisierung von Biomolekülen umfassen.

Das erfindungsgemäße Verfahren sieht gemäß Anspruch 8 vor, daß eine Probe in Kontakt mit einem Festphasensubstrat gebracht wird, das mindestens einen Bindungsbereich aufweist, der zur Immobilisierung von Biomolekülen geeignet ist, und bei dem die Immobilisierung in Gegenwart einer Substanz erfolgt, die in der Lage ist, die dreidimensionale Konformation der Biomoleküle zu stabilisieren, wobei das Substrat im Bindungsbereich reaktive Bindungsstellen aufweist und als Substanz Polyole eingesetzt werden, die im Bindungsbereich des Festphasensubstrats mittels kovalenter Bindungen an einen Teil der Bindungsstellen gebunden werden.

In einer bevorzugten Ausgestaltung des Verfahren ist vorgesehen, daß es sich bei den zu immobilisierenden Biomolekülen um Proteine handelt. Die reaktiven Bindungsstellen im Bindungsbereich müssen daher für die kovalente Bindung von Proteinen geeignete funktionelle Gruppen aufweisen.

In weiteren erfindungsgemäßen Ausgestaltungen kann jedoch auch vorgesehen sein, daß es sich bei den zu immobilisierenden Biomolekülen z.B. um Zucker, Lipide oder Nukleinsäuren handelt. Die reaktiven Bindungsstellen im Bindungsbereich können die jeweils für diese Fälle geeigneten, dem Fachmann bekannten, funktionellen Gruppen aufweisen.

Bekannte Verfahren zur Immobilisierung von Biomolekülen, bei denen als Biomoleküle beispielsweise Proteine verwendet werden, umfassen im wesentlichen folgende Schritte:
1. Eine proteinhaltige Lösung wird in Kontakt mit einem aktivierten Substrat gebracht. Bei dem Substrat kann es sich z.B. um ein Filter, eine Membran, ein Chip, ein Reaktionsgefäß handeln, um nur einige übliche Einrichtungen aufzuzählen.
   Übliche Substrate weisen reaktive Bindungsstellen auf, die mit funktionellen Gruppen der zu bindenden Proteine kovalente Bindungen eingehen können. Bei Kontakt mit dem Substrat werden die Proteine kovalent an die reaktiven Bindungsstellen gebunden und auf diese Weise an dem Substrat immobilisiert.
2. Das Substrat wird dann ggf. gewaschen.
3. In einem nächsten Schritt werden die Bindungsstellen auf dem Substrat durch z.B. Zugabe eines starken nucleophilen Agens, z.B. eines kurzkettiges Amins, blockiert.
4. Es wird dann erneut gewaschen, wobei dieser letzte Waschpuffer eine hohe Konzentration einer Schutzsubstanz enthält, die in der Lage ist, die dreidimensionale Konformation von Proteinen zu stabilisieren. In bekannten Verfahren ist diese Substanz z.B. das Polyol Trehalose.
5. Das Substrat wird dann in üblicher Weise getrocknet, wobei die Trehalose aus dem Waschpuffer auf das Substrat adsorbiert wird.

Im Gegensatz zu diesen Verfahren aus dem Stand der Technik entfallen bei dem erfindungsgemäßen Verfahren der Blockierungsschritt mit einem starken nucleophilen Agens und der letzte Waschschritt, bei dem ein Polyol auf das Substrat adsorbiert wird. Anders als im Stand der Technik wird das Polyol bei dem erfindungsgemäßen Verfahren überdies nicht auf die Substratoberfläche adsorbiert, sondern geht mit dieser Oberfläche genau wie die Biomoleküle eine kovalente Bindung ein.

Die kovalente Immobilisierung der Polyole auf dem Substrat hat keinen nachteiligen Einfluß auf ihre Funktion als Schutzsubstanz für die Biomoleküle. Wesentlicher Vorteil und Unterschied gegenüber den bereits bekannten Verfahren ist jedoch, daß die Polyole aufgrund ihrer festen Kopplung an dem Substrat eine spätere Anwendung nicht mehr stören. Vor einer Aufarbeitung des Substrates mit den daran immobilisierten Biomolekülen muß also kein aufwändiger Waschschritt mehr vorgesehen werden.

In einer bevorzugten Ausgestaltung des erfmdungsgemäßen Verfahrens ist vorgesehen, daß die Polyole kovalent über im Bindungsbereich angeordnete Polymerketten, die die reaktiven Bindungsstellen tragen, am Festphasensubstrat gebunden werden. Die Verwendung solcher Polymerketten erhöht die aktive Oberfläche des Substrats und gestattet so die Bindung von mehr Polyolen und/oder Biomolekülen.

Besonders bevorzugt werden die Polyole und die Biomoleküle gleichzeitig am Festphasensubstrat gebunden. Am einfachsten läßt sich dies erreichen, indem man z.B. eine zur Immobilisierung eingesetzte Proteinlösung mit dem Polyol versetzt. In Abhängigkeit von der eingesetzten Polyolkonzentration kommt es dann zu einer entsprechenden anteiligen Kopplung der Bindungsstellen mit der Polyolen und den Biomolekülken bzw. Proteinen.

In einer Lösung, die als Biomoleküle z.B. Proteine enthält, liegen geeignete Polyolkonzentrationen zwischen 1 und 100 g/l. Besonders geeignet sind Polyolkonzentrationen zwischen 5 und 50 g/l.

In einer weiteren, besonders bevorzugten Ausgestaltung des Verfahrens ist vorgesehen, daß das eingesetzte Festphasensubstrat bereits vorkonfektioniert gebundene Polyole enthält.

Ein solches, vorkonfektioniert mit kovalent gebundenen Polyolen augestattetes Festphasensubstrat eignet sich insbesondere für das Bespotten mit Protein- oder Nukleinsäuresonden bzw. -proben. Mögliche Anwendungsbereiche sind z.B. die großserienhafte Herstellung von standardisierten Bioarrays, bei denen die immobilisierten Biomoleküle während der Lagerung der Bioarrays bis zu deren Benutzung vor Denaturierung geschützt werden müssen.

In einer bevorzugten Ausgestaltung des Verfahrens ist vorgesehen, daß das Festphasensubstrat, nachdem es mit der Probe in Kontakt gebracht wurde, getrocknet wird. In dieser Ausgestaltung kommt der schützende Effekt der als Schutzsubstanz verwendeten Polyole besonders zum Tragen, da die Trocknung eines mit Biomolekülen beschichteten Substrats, das keine Polyole aufweist, in der Regel zur Denaturierung der Biomoleküle führt.

In einer weiteren bevorzugten Ausgestaltung des Verfahrens ist vorgesehen daß die Polymerketten im Bindungsbereich zusätzlich PEG aufweisen. Solche PEGenthaltenen Polymere schirmen die Substratoberfläche z.B. gegen unspezifische Absorptionen anderer Biomoleküle ab.

Im Rahmen der Erfindung einsetzbare Polyole können z.B. Mono-, Di- oder Trisaccharide sein. Geeignet sind z.B. Maltose, Saccharose, Raffinose, Galaktose oder Glukose. Die obigen Aufzählungen sind nur beispielhaft und keinesfalls abschließend. Grundsätzlich sind alle Substanzen geeignet, die über geeignete funktionelle Gruppen, wie z.B. OH-Gruppen, an eine aktivierte Oberfläche eines Substrats gekoppelt werden können und die in der Lage sind, die dreidimensionale Konformation von Biomolekülen, insbesondere von Proteinen, zu stabilisieren. Besonders bevorzugt wird das Polyol Trehalose eingesetzt, das, wie Untersuchungen der Anmelderin ergeben haben, die auf dem Substrat immobilisierten Biomoleküle sehr wirksam stabilisiert.

In einer bevorzugten Ausgestaltung des Verfahrens ist vorgesehen, daß das Festphasensubstrat ein Biochip, ein Enzymchip, ein Proteinarray, eine Filtermembran, ein Microbead, ein Reaktionsgefäß, ein Mikrokanalsystem, ein Durchflußschlauchsystem, eine Pipettenspitze oder eine Durchflußkanüle ist.

Einige der erfindungsgemäß eingesetzten Substrate können generell nach der z.B. von Ulbricht et al. in Colloids and Surfaces Vol. 138, 1998, S. 353, beschriebenen in-situ-Technik mittels Photo-initiierter-Pfropf-Polymerisation hergestellt werden.

Zur Herstellung der erfindungsgemäßen Substrate werden bei der erwähnten Methode Monomere, an die Polyole gebunden sind, mit Monomeren, die die Bindungsstellen für die Proteine, z.B. esteraktive Gruppen, aufweisen copolymerisiert. Durch Einstellung der Ausgangskonzentrationen der unterschiedlichen Monomere kann man ein gewünschtes Verhältnis zwischen Polyol- und Proteinbindungsstellen in den Polymeren auf besonders einfache Weise einstellen. Denkbar ist natürlich, daß man noch weitere Monomere mit anderen Gruppen in die Polymere einpolymerisiert. Z.B. könnte man Monomere copolymerisieren, die PEG enthalten.

Im folgenden soll die Erfindung anhand von einem Beispiel näher erläutert werden. Das Beispiel betrifft ein im Rahmen der Erfindung durchführbares Protokoll, bei dem ein Protein und Trehalose gleichzeitig mit dem Substrat in Kontakt gebracht werden.

### Beispiel: Immobilisierung von Trypsin an carboxylierte Nylonmembranen

Um mit aktivem Trypsin beschichtete Membranen zu erhalten, wie sie z.B. in der "Double Parallel Digestion"-Methode (Bienvenut et al. in Anal Chem Vol. 71, 1999, S. 4800-4807) zur Proteinidentifikation benutzt werden, und die darüber hinaus trockenbar sein sollen, wird wie folgt vorgegangen:
Verwendete Abkürzungen:
   PP: Phosphatpuffer (0,1M, pH 4,8)
   PVDF: Polyvinylidenfluorid
   EDC: 1-Ethyl-3-(3-dimethylaminopropyl)carbodiimid
   NHS: N-Hydoxysuccinimid
   PBS: Phosphatgepufferte physiologische Salzlösung (pH 7,4) Triton X: nichtionisches Detergens
   Tween: nichtionisches Detergens

1. Eine carboxylierte PVDF-Membran (Millipore, Inc.) mit einer mittleren Porengröße von 0,45µm wird 10 min mit PP inkubiert und sodann mit in PP gelöstem EDC (25mg/ml) sowie in PP gelöstem NHS (10mg/ml) im Verhältnis 1 : 1 für 12 min aktiviert.
2. Anschließend wird die aktivierte Membran mit PP gewaschen, und für 120 min mit einer PP-Lösung inkubiert, die Trypsin (2µg/100µl) sowie Trehalose (50g/l) enthält. Anschließend wird mit PP gewaschen.
3. Die Membran wird dann mit 20µg/100µl Methoxyethylamin für 10 min inkubiert, um Trypsin und Trehalose kovalent an die Membran zu binden.
4. Anschließend wird 3x mit PP, der 0,05% Triton X enthält, gewaschen, und mit PBS/Tween für 10 min inkubiert.
5. Die letzte Lösung wird dann entfernt und die Membran getrocknet.

Die nach dieser Methode mit aktivem Trypsin beladene Membran ist im trockenen Zustand lagerstabil und kann auf einfache Weise transportiert oder verschickt werden.

## Patentansprüche

1. Festphasensubstrat mit mindestens einem Bindungsbereich, der zur Immobilisierung von Biomolekülen geeignet ist, **dadurch gekennzeichnet, dass** das Substrat im Bindungsbereich reaktive Bindungsstellen aufweist, wobei an einem Teil dieser Bindungsstellen Polyol mittels kovalenter Bindungen immobilisiert ist und wobei an einem Teil dieser Bindungsstellen Biomoleküle mittels kovalenter Bindungen immobilisiert sind.

2. Festphasensubstrat gemäß Anspruch 1, **dadurch gekennzeichnet, dass** es sich bei den Biomolekülen um Proteine handelt.

3. Festphasensubstrat gemäß Anspruch 1 oder 2, **dadurch gekennzeichnet, dass** im Bindungsbereich Polymerketten angeordnet sind, die die reaktiven Bindungsstellen tragen.

4. Festphasensubstrat gemäß Anspruch 3, **dadurch gekennzeichnet, dass** in den Polymerketten zusätzlich PEG gebunden ist.

5. Festphasensubstrat gemäß einem der vorhergehenden Ansprüche, **dadurch gekennzeichnet, dass** das eingesetzte Polyol ein Mono-, Di- oder Trisaccharid ist.

6. Festphasensubstrat gemäß einem der vorhergehenden Ansprüche, **dadurch gekennzeichnet, dass** das eingesetzte Polyol Trehalose ist.

7. Festphasensubstrat gemäß einem der vorhergehenden Ansprüche, **dadurch gekennzeichnet, dass** das Festphasensubstrat ein Biochip, ein Enzymchip, ein Proteinarray, eine Filtermembran, ein Microbead, ein Reaktionsgefäß, ein Mikrokanalsystem, ein Durchflußschlauchsystem, eine Pipettenspitze oder eine Durchflußkanüle ist.

8. Verfahren zur Immobilisierung von Biomolekülen in einer Probe, bei dem die Probe in Kontakt mit einem Festphasensubstrat gebracht wird, das mindestens einen Bindungsbereich aufweist, der zur Immobilisierung von Biomolekülen geeignet ist, und bei dem die Immobilisierung in Gegenwart einer Substanz erfolgt, die in der Lage ist, die dreidimensionale Konformation der Biomoleküle zu stabilisieren, **dadurch gekennzeichnet, dass** das Substrat im Bindungsbereich reaktive Bindungsstellen aufweist, wobei während des Verfahrens an einen Teil dieser Bindungsstellen Polyol mittels kovalenter Bindungen immobilisiert wird und wobei an einen Teil dieser Bindungsstellen Biomoleküle mittels kovalenter Bindungen immobilisiert werden.

9. Verfahren gemäß Anspruch 8, **dadurch gekennzeichnet, dass** es sich bei den Biomolekülen um Proteine handelt.

10. Verfahren gemäß Anspruch 8 oder 9, **dadurch gekennzeichnet, dass** die Polyole kovalent über im Bindungsbereich angeordnete Polymerketten, die die reaktiven Bindungsstellen tragen, am Festphasensubstrat gebunden werden.

11. Verfahren gemäß einem der Ansprüche 8 - 10, **dadurch gekennzeichnet, dass** Polyole und Biomoleküle gleichzeitig am Festphasensubstrat gebunden werden.

12. Verfahren gemäß einem der Ansprüche 8 - 10, **dadurch gekennzeichnet, dass** ein Festphasensubstrat eingesetzt wird, das bereits gebundene Polyole enthält.

13. Verfahren gemäß einem der Ansprüche 8 - 12, **dadurch gekennzeichnet, dass** das Festphasensubstrat, nachdem es mit der Probe in Kontakt gebracht wurde, getrocknet wird.

14. Verfahren gemäß einem der Ansprüche 8 - 13, **dadurch gekennzeichnet, dass** die Polymerketten im Bindungsbereich zusätzlich PEG aufweisen.

15. Verfahren gemäß einem der Ansprüche 8 - 14, **dadurch gekennzeichnet, dass** das eingesetzte Polyol ein Mono-, Di- oder Trisaccharid ist.

16. Verfahren gemäß einem der Ansprüche 8 - 15, **dadurch gekennzeichnet, dass** das eingesetzte Polyol Trehalose ist.

17. Verfahren gemäß einem der Ansprüche 8 - 16, **dadurch gekennzeichnet, dass** das Festphasensubstrat ein Biochip, ein Enzymchip, ein Proteinarray, eine Filtermembran, ein Microbead, ein Reaktionsgefäß, ein Mikrokanalsystem, ein Durchflußschlauchsystem, eine Pipettenspitze oder eine Durchflußkanüle ist.

18. Verwendung eines Festphasensubstrats gemäß einem der Ansprüche 1 bis 7 als Slide zum Spotten von Biomolekülen.

## Claims

1. Solid-phase substrate having at least one binding region which is suitable fo r immobilizing biomolecules, **characterized in that** the substrate comprises reactive binding sites in the binding region, wherein polyol is immobilized to part of said binding sites by means of covalent bonds and wherein biomolecules are immobilized to part of said binding sites by means of covalent bonds.

2. Solid-phase substrate according to Claim 1, **characterized in that** the biomolecules are proteins.

3. Solid-phase substrate according to Claim 1 or 2, **characterized in that** polymer chains carrying the reactive binding sites are arranged in the binding region.

4. Solid-phase substrate according to Claim 3, **characterized in that** PEG is additionally bonded in the polymer chains.

5. Solid-phase substrate according to any of the preceding claims, **characterized in that** the polyol used is a monosaccharide, disaccharide or trisaccharide.

6. Solid-phase substrate according to any of the preceding claims, **characterized in that** the polyol used is trehalose.

7. Solid-phase substrate according to any of the preceding claims, **characterized in that** the solid-phase substrate is a biochip, an enzyme chip, a protein array, a filter membrane, a microbead, a reaction vessel, a microchannel system, a flow-through tube system, a pipette tip or a flow-through cannula.

8. Process for immobilizing biomolecules in a sample, wherein the sample is contacted with a solid-phase substrate which comprises at least one binding region which is suitable fo r immobilizing biomolecules, and wherein the immobilization is achieved in the presence of a substance which is capable of stabilizing th e three-dimensional conformation of the biomolecules, **characterized in that** the substrate comprises reactive binding sites in the binding region, wherein, during the process, polyol is immobilized to part of said binding sites by means of covalent bonds and wherein biomolecules are immobilized to part of said binding sites by means of covalent bonds.

9. Process according to Claim 8, **characterized in that** the biomolecules are proteins.

10. Process according to Claim 8 or 9, **characterized in that** the polyols are covalently bonded to the solid-phase substrate via polymer chains which carry the reactive binding sites and are arranged in the binding region.

11. Process according to any of Claims 8-10, **characterized in that** polyols and biomolecules are bonded simultaneously to th e solid-phase substrate.

12. Process according to any of Claims 8-10, **characterized in that** use is made of a solid-phase substrate which already contains bonded polyols.

13. Process according to any of Claims 8-12, **characterized in that** the solid-phase substrate is dried after it has been contacted with the sample.

14. Process according to any of claims 8-13, **characterized in that** the polymer chains in the binding region additionally comprise PEG.

15. Process according to any of Claims 8-14, **characterized in that** the polyol used is a monosaccharide, disaccharide or trisaccharide.

16. Process according to any of Claims 8-15, **characterized in that** the polyol us e d is trehalose.

17. Process according to any of Claims 8-16, **characterized in that** the solid-phase substrate is a biochip, an enzyme chip, a protein array, a filter membrane, a microbead, a reaction vessel, a microchannel system, a flow-through tube system, a pipette tip or a flow-through cannula.

18. Use of a solid-phase substrate according to any of Claims 1 to 7 as a slide for spotting biomolecules.

## Revendications

1. Substrat en phase solide comprenant au moins une zone de liaison appropriée pour l'immobilisation de biomolécules, **caractérisé en ce que** le substrat comprend des emplacements de liaison réactifs dans la zone de liaison, du polyol étant immobilisé par des liaisons covalentes sur une partie de ces emplacements de liaison et des biomolécules étant immobilisées par des liaisons covalentes sur une partie de ces emplacements de liaison.

2. Substrat en phase solide selon la revendication 1, **caractérisé en ce que** les biomolécules sont des protéines.

3. Substrat en phase solide selon la revendication 1 ou 2, **caractérisé en ce que** des chaînes polymères qui portent les emplacements de liaison réactifs sont placées dans la zone de liaison.

4. Substrat en phase solide selon la revendication 3, **caractérisé en ce que** du PEG est également relié dans les chaînes polymères.

5. Substrat en phase solide selon l'une quelconque des revendications précédentes, **caractérisé en ce que** le polyol utilisé est un mono-, di- ou trisaccharide.

6. Substrat en phase solide selon l'une quelconque des revendications précédentes, **caractérisé en ce que** le polyol utilisé est le tréhalose.

7. Substrat en phase solide selon l'une quelconque des revendications précédentes, **caractérisé en ce que** le substrat en phase solide est une biopuce, une puce à enzymes, une puce à protéines, une membrane de filtration, une microbille, un récipient de réaction, u n système à microcanal, un système de tube d'écoulement, une pointe de pipette ou une canule d'écoulement.

8. Procédé d'immobilisation de biomolécules dans un échantillon, selon lequel l'échantillon est mis en contact avec un substrat en phase solide qui comprend au moins une région de liaison appropriée pour l'immobilisation de biomolécules et selon lequel l'immobilisation a lieu en présence d'une substance capable de stabiliser la conformation tridimensionnelle des biomolécules, **caractérisé en ce que** le substrat comprend des emplacements de liaison réactifs dans la zone de liaison, du polyol étant immobilisé par des liaisons covalentes sur une partie de ces emplacements de liaison et des biomolécules étant immobilisées par des liaisons covalentes sur une partie de ces emplacements de liaison pendant le procédé.

9. Procédé selon la revendication 8, **caractérisé en ce que** les biomolécules sont des protéines.

10. Procédé selon la revendication 8 ou 9, **caractérisé en ce que** les polyols sont reliés au substrat en phase solide par des liaisons covalentes par le biais de chaînes polymères placées dans la zone de liaison, qui portent les emplacements de liaison réactifs.

11. Procédé selon l'une quelconque des revendications 8 à 10, **caractérisé en ce que** les polyols et les biomolécules sont reliés simultanément au substrat en phase solide.

12. Procédé selon l'une quelconque des revendications 8 à 10, **caractérisé en ce qu'**un substrat en phase solide qui contient déjà des polyols reliés est utilisé.

13. Procédé selon l'une quelconque des revendications 8 à 12, **caractérisé en ce qu** e le substrat en phase solide est séché après avoir été mis en contact avec l'échantillon.

14. Procédé selon l'une quelconque des revendications 8 à 13, **caractérisé en ce qu** e les chaînes polymères dans la zone de liaison comportent également du PEG.

15. Procédé selon l'un e quelconque des revendications 8 à 14, **caractérisé en ce que** le polyol utilisé est un mono-, di- ou trisaccharide.

16. Procédé selon l'une quelconque des revendications 8 à 15, **caractérisé en ce que** le polyol utilisé est le tréhalose.

17. Procédé selon l'une quelconque des revendications 8 à 16, **caractérisé en ce qu** e le substrat en phase solide est une biopuce, une puce à enzymes, une puce à protéines, une membrane de filtration, une microbille, un récipient de réaction, u n système à microcanal, un système d e tube d'écoulement, une pointe de pipette ou une canule d'écoulement.

18. Utilisation d'un substrat en phase solide selon l'une quelconque des revendications 1 à 7 en tant que lame pour l'observation de biomolécules.
